# EUROPÄISCHE PATENTSCHRIFT

(11) **EP 1 270 714 B1**
(45) Veröffentlichungstag und Bekanntmachung des Hinweises auf die Patenterteilung: **03.01.2007**
(21) Anmeldenummer: 02013371.6
(22) Anmeldetag: 19.06.2002
(51) Int. Cl.: C12M 1/107, C12M 1/02

(54) **Biogasanlage**
Biogas producing installation
Installation de production de biogaz

(30) Priorität: 29.06.2001 DE 20110792 U
(43) Veröffentlichungstag der Anmeldung: 02.01.2003
(73) Patentinhaber: U.T.S. Umwelt-Technik-Süd GmbH, 84419 Obertaufkirchen (DE)
(72) Erfinder: Bürger, Adam, 84405 Gr-ntegernbach (DE)
(74) Vertreter: Neubauer, Hans-Jürgen

(56) Entgegenhaltungen:
- EP-A- 0 197 903
- DE-A- 4 120 988
- DE-A- 19 951 959
- DE-C- 4 015 478
- DE-C- 19 732 198

## Beschreibung

Die Erfindung betrifft eine Biogasanlage zur Fermentation von organischen Stoffen nach dem Oberbegriff des Anspruchs 1.

In derartigen Biogasanlagen läuft ein Fermentationsprozess ab, bei dem organische Stoffe, wie z.B. Gras, Stalldung, Jauche, Klärschlamm, Stroh und dergleichen vergast werden. Dieser Fermentationsprozess findet in einem Fermenterbehälter statt, in dem die organischen Stoffe mit Flüssigkeit versetzt werden und der Fermentations- bzw. Vergasungsprozess unter aeroben oder anaeroben Bedingungen durch Mikroorganismen, wie z.B. Hefen, Bakterien, etc. durchgeführt wird. Die bei der Fermentation entstehenden Biogase sammeln sich in einem oberen Fermenterbehälterbereich und können direkt zur Energieerzeugung verwendet werden, z.B. als Heizgas zur Stromerzeugung in nachgeschalteten Brennkraftmaschinen mit Elektrogeneratoren.

Solche Biogasanlagen zur Fermentation von organischen Stoffen finden meist in der dezentralen Verarbeitung und Verwertung von organischen Reststoffen im landwirtschaftlichen Bereich Verwendung. Die Biogasanlagen sind hier insbesondere aus wirtschaftlichen und umweltschutztechnischen Gründen erstrebenswert, da dadurch für den Transport der organischen Reststoffe aus Haushalten und landwirtschaftlichen Betrieben nur kurze Transportwege erforderlich sind. Große und geruchsintensive Deponien werden dadurch entbehrlich und die landwirtschaftlichen sowie haushaltlichen organischen Reststoffe können direkt vor Ort zur Energieerzeugung verwendet werden. Die in der dezentralen Rohstoffverwertung eingesetzten Fermenter weisen einen Durchmesser von bis zu 20 m, in der Regel von ungefähr 5 bis 15 m, und eine Höhe von 3 bis 8 m auf, wobei die Fermenter entweder im Erdboden eingelassen oder als Hochbehälter ausgeführt sind.

Für eine gleichmäßige Verteilung der Feststoffe in der Schlämmung und für das Verhindern des Festsetzens der Feststoffe an den Fermenterseitenwänden ist ein Rührwerk als Umwälzeinrichtung im Fermenterbehälter erforderlich. Eine gattungsgemäße Biogasanlage zur Fermentation von organischen Stoffen mit einem Fermenter, der einen Fermenterbehälter und ein Rührwerk umfasst, ist aus der DE 197 14 342 C2 bekannt. Der Fermenterbehälter ist hier aus einer Fermenterbodenwand, Fermenterseitenwänden und einer Fermenterdeckenwand aufgebaut. Zudem ist ein Tauchrührgerät als Rührwerk über eine Führungseinrichtung an einer Stützstange höhenverstellbar gehalten. Für diese Höhenverstellung ist eine Seilwinde am oberen Stützstangenbereich sowie eine kraftübertragende Seilverbindung zum Tauchrührgerät vorgesehen. Ferner enthält die Fermenterdeckenwand eine Wartungsöffnung und einen darüber angebrachten Dom, durch die die Stützstange nach oben dicht herausgeführt ist. Durch eine Verschwenkung der Stützstange um die Stangenachse sind zudem unterschiedliche Winkelstellungen des Tauchrührgeräts möglich.

Die vorstehende höhenverstellbare und winkelverstellbare Anordnung eines Tauchrührgeräts an einer zentralen Stützstange im Fermenterbehälter ergibt zwar eine gute Rühr- und Umwälzfunktion, ist jedoch relativ aufwändig und teuer. Insbesondere ist eine solche Anordnung vorzugsweise für größere Biogasanlagen in Verbindung mit einem Bodenräumgerät geeignet. Zudem kann die Stützstange im Bereich des Tauchrührgeräts den Strömungswiderstand ungünstig erhöhen. Weiter kann die Kabelzuführung zum Elektroantrieb des Tauchrührgeräts Probleme verursachen, da das Kabel nicht fest verlegt werden kann und zusammen mit dem Tauchrührgerät entlang der Stützstange beweglich gehalten sein muss.

Aufgabe der Erfindung ist es demgegenüber, eine gattungsgemäße Biogasanlage zur Fermentation von organischen Stoffen so weiterzubilden, dass mit einer alternativen Anordnung eines Tauchrührgeräts bei guter Rühr- und Umwälzfunktion eine kostengünstigere Lösung, insbesondere für kleinere Biogasanlagen, erreichbar ist.

Diese Aufgabe wird mit den Merkmalen des Anspruchs 1 gelöst.

Gemäß Anspruch 1 ist die Halte- und Stelleinrichtung für das wenigstens eine Tauchrührgerät als Schwingenanordnung ausgebildet. Dazu ist wenigstens ein Schwingen-Gelenklager mit etwa horizontaler Gelenklagerachse ortsfest im Fermenterbehälter angebracht. Mit diesem Schwingen-Gelenklager ist ein Schwingenarm schwenkbar verbunden. Das Tauchrührgerät ist im Bereich des freien Schwingenarmendes angebracht, so dass bei unterschiedlichen Schwenkpositionen des Schwingenarms entsprechend zugeordnete unterschiedliche Höhenpositionen des Tauchrührgeräts einstellbar sind.

Damit ist weder eine aufwändige Stützstange noch eine aufwändige Führungseinrichtung zwischen Tauchrührgerät und Stützstange erforderlich. Ein einfaches Schwingen-Gelenklager als Schwenklager ist demgegenüber wesentlich einfacher und kostengünstiger herstellbar und montierbar. Zudem ist der Schwingenarm zwar stabil auszuführen, jedoch hinsichtlich evtl. Toleranzen unkritisch im Gegensatz zu der Führungseinrichtung an der Stützstange nach dem Stand der Technik. Vorteilhaft hängt das Tauchrührgerät am Schwingenarmende sehr frei im Fermenterbehälter, so dass von dieser Halterung des Tauchrührgeräts nur eine vorteilhaft geringe Erhöhung des Strömungswiderstands ausgeht. Bei größeren Fermenterbehältern werden gegebenenfalls zwei oder mehrere Tauchrührgeräte verwendet, die zweckmäßig gegenüberliegend angebracht sind.

Je nach den Gegebenheiten kann das Tauchrührgerät in geeigneten Winkelstellungen der Rührachse fest am Schwingenarmende angebracht sein. Eine für übliche Anwendungen gut geeignete Anordnung nach Anspruch 2 besteht darin, dass das Tauchrührgerät drehangetriebene Rührelemente aufweist und so am Schwingenarm angebracht ist, dass die Rührelementdrehachse achsparallel zur Gelenklagerachse verläuft. Die Rührelement-Drehachse verläuft somit in allen Höheneinstellungen etwa waagrecht und etwa in einem rechten Winkel zum Schwingenarm, so dass im wesentlichen eine Umwälzung in einer waagrechten Höhenschicht des Fermenterbehälters entsprechend der Höheneinstellung des Tauchrührgeräts erfolgt. Als Drehantriebe können elektrische, hydraulische oder pneumatische Antriebe eingesetzt werden.

Grundsätzlich kann der Schwingenarm unterschiedliche geometrische Strukturen aufweisen. Eine kostengünstig herstellbare und stabile Ausführung wird nach Anspruch 3 erreicht, wenn der Schwingenarm als rechteckiger Schwingenrahmen ausgeführt ist. Dabei ist ein Rahmenteil an zwei beabstandeten, koaxial angeordneten Schwingen-Gelenklagern schwenkbar gelagert und am gegenüberliegenden Rahmenteil ist das Tauchrührgerät befestigt. Durch den Rechteckrahmen wird eine stabile Kraftabstützung der Reaktionskräfte des Tauchrührgeräts bei geringem Material- und Kostenaufwand erreicht.

Wenn das Tauchrührgerät in an sich bekannter Weise einen zylindrisch geformten Drehantrieb, insbesondere einen Elektrodrehantrieb aufweist, kann dieser in einer kompakten und stabilen Anordnung zweckmäßig mit dem vorstehend bezeichneten, gegenüberliegenden Rahmenteil in einer Parallelanordnung fest, jedoch für Wartungszwecke lösbar, verbunden werden, wobei entlang des Rahmenteils geeignete Anbindungspunkte geschaffen werden können. Wenn als Rührelement des Tauchrührgeräts in an sich bekannter Weise ein Rührpropeller mit wenigstens zwei Rührflügeln verwendet ist, kann der Rührpropeller den Schwingenrahmen seitlich überragen, wo er ohne Behinderung durch den Schwingenrahmen frei drehen kann.

Ein besonderer Vorteil der vorstehenden Schwingenanordnung besteht auch darin, dass gemäß Anspruch 5 eine Versorgungsleitung, z. B. ein Elektrokabel, zu einem Elektroantrieb des Tauchrührgeräts ortsfest bis zum Schwingen-Gelenklager und wiederum ortsfest am Schwingenarm entlang zum Elektrodrehantrieb geführt werden kann, wobei in Verbindung mit einer Höhenverstellung das Elektrokabel lediglich im Bereich des Schwingen-Gelenklagers eine Schwenkbewegung durchführen muss. Damit ist die Führung des Elektrokabels unproblematisch.

Grundsätzlich ist mit der vorbeschriebenen Schwingenanordnung bei jeder Positionierung des Schwingen-Gelenklagers im Fermenterbehälter eine Höhenverstellung eines Tauchrührgeräts zu erreichen. Eine zweckmäßige Anordnung nach Anspruch 6 ergibt sich, wenn das Schwingen-Gelenklager im Bereich unmittelbar unter der Fermenterdeckenwand angebracht ist, wobei zudem nach Anspruch 7 für einfache Montageverhältnisse und gute Rührergebnisse eine achswandparallele Anbringung an einer Fermenterseitenwand vorteilhaft ist.

Jedenfalls ist es von besonderem Vorteil, wenn gemäß Anspruch 8 das wenigstens eine Schwingen-Gelenklager so angeordnet ist und die Länge des Schwingenarms so gewählt ist, dass eine Verstellung des Tauchrührgeräts unmittelbar in den Bereich der Fermenterdeckenwand durchführbar ist. Dort kann dann auf einfache Weise eine Wartungsposition für das Tauchrührgerät geschaffen werden, wobei das Tauchrührgerät für Wartungszwecke durch eine dortige dicht verschließbare Wartungsöffnung in der Fermenterdeckenwand zugänglich ist. Diese Wartungsöffnung soll wenigstens so groß sein, dass das Tauchrührgerät durch die Öffnung hindurch montierbar und demontierbar ist.

Für die Verstellung des Tauchrührgeräts ist eine Betätigungseinrichtung erforderlich, mit der einerseits die Verstellung durchführbar und andererseits eine Festlegung in der jeweiligen, verstellten Position möglich ist. In einer besonders einfachen Ausführung gemäß Anspruch 9 kann als Betätigungseinrichtung ein Stellgestänge verwendet werden, das mit dem Schwingenarm und/oder dem Tauchrührgerät in einem Abstand zum Schwingen-Gelenklager gelenkig verbunden ist und dicht über eine Dichtmanschette in den Außenbereich des Fermenterbehälters geführt ist. Durch eine Zug-/Druckverstellung des Stellgestänges erfolgt damit auch eine entsprechende Schwingenarmverstellung, wobei das Stellgestänge in seiner Position außerhalb des Fermenterbehälters durch an sich bekannte Rasten-, Klinken- und/oder Klemmmechanismen feststellbar ist.

In einer alternativen Ausführungsform einer Betätigungseinrichtung kann auch ein für diese Zwecke an sich bekanntes Betätigungsseil, vorzugsweise in Verbindung mit einer Seilwinde, verwendet werden, wobei das Betätigungsseil mit dem Schwingenarm und/oder dem Tauchrührgerät verbunden und über Umlenk- und Dichtelemente dicht in den Außenbereich des Fermenterbehälters geführt ist.

Insbesondere bei großen und tiefen Fermenterbehältern kann es gemäß Anspruch 11 zweckmäßig sein, eine an sich bekannte weitere Möglichkeit einer Höhenverstellung an einer vertikalen Höhenstellschiene zu schaffen.

Anhand einer Zeichnung wird die Erfindung näher erläutert.

Es zeigen:
- Fig. 1: eine schematische Darstellung einer Biogasanlage mit einer ersten Ausführungsform eines über einen Schwingenarm im Fermenterbehälter angeordneten Tauchrührgeräts,
- Fig. 2: eine alternative Ausführungsform zu der Darstellung gemäß Fig. 2,
- Fig. 3: eine weitere alternative Ausführungsform zu den Darstellungen gemäß den Figuren 1 und 2,
- Fig. 4: eine vergrößerte Detaildarstellung einer Betätigungseinrichtung für eine Ausführungsform entsprechend Fig. 1, und
- Fig. 5: eine vergrößerte Draufsicht auf die Anordnung entsprechend der Ausführungsform gemäß Fig. 1.

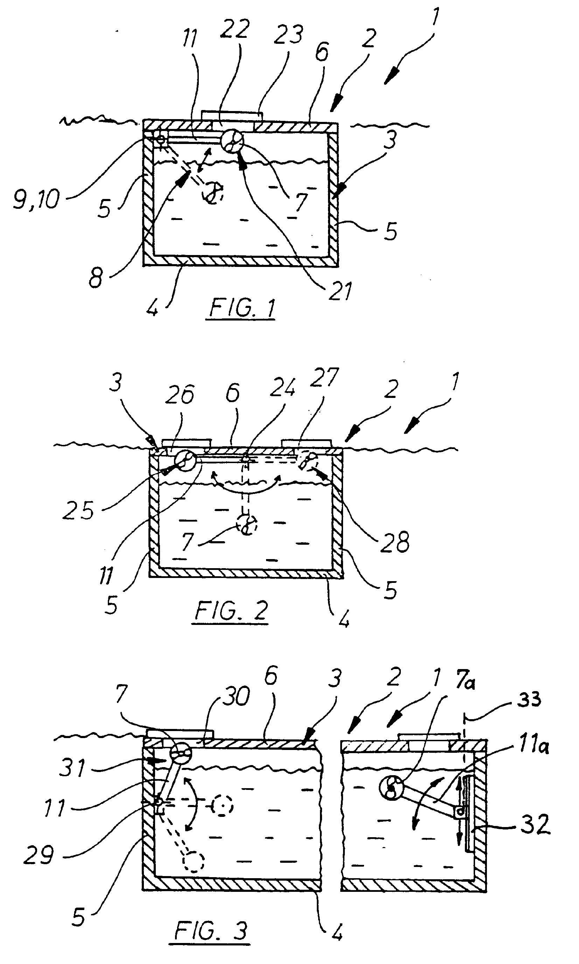

In der Fig. 1 ist schematisch ein Teilbereich einer Biogasanlage 1 zur Fermentation von organischen Stoffen gezeigt, die einen Fermenter 2 mit einem Fermenterbehälter 3 umfasst. Dieser Fermenterbehälter 3 weist eine Fermenterbodenwand 4, Fermenterseitenwände 5 und eine Fermenterdeckenwand 6 auf.

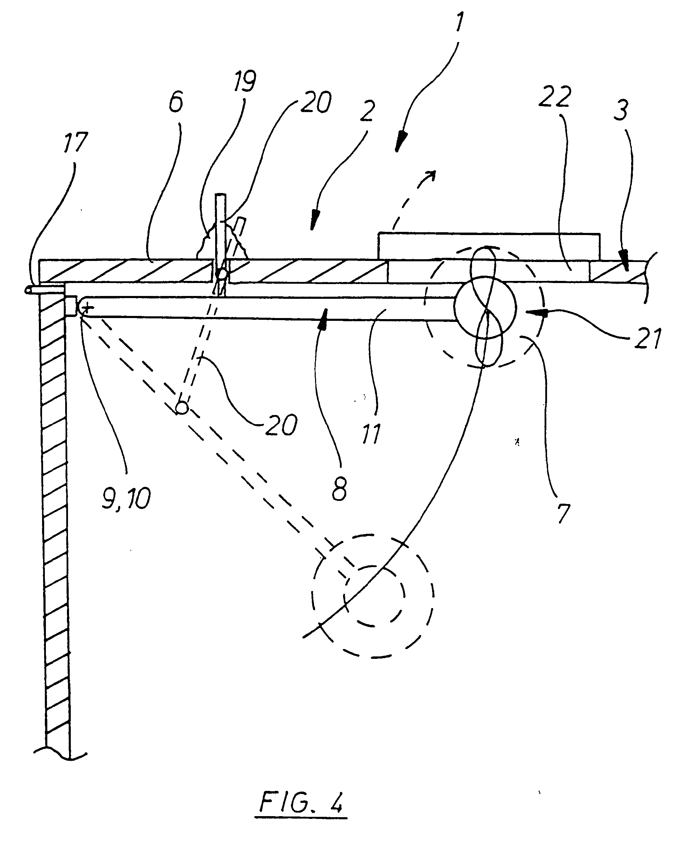

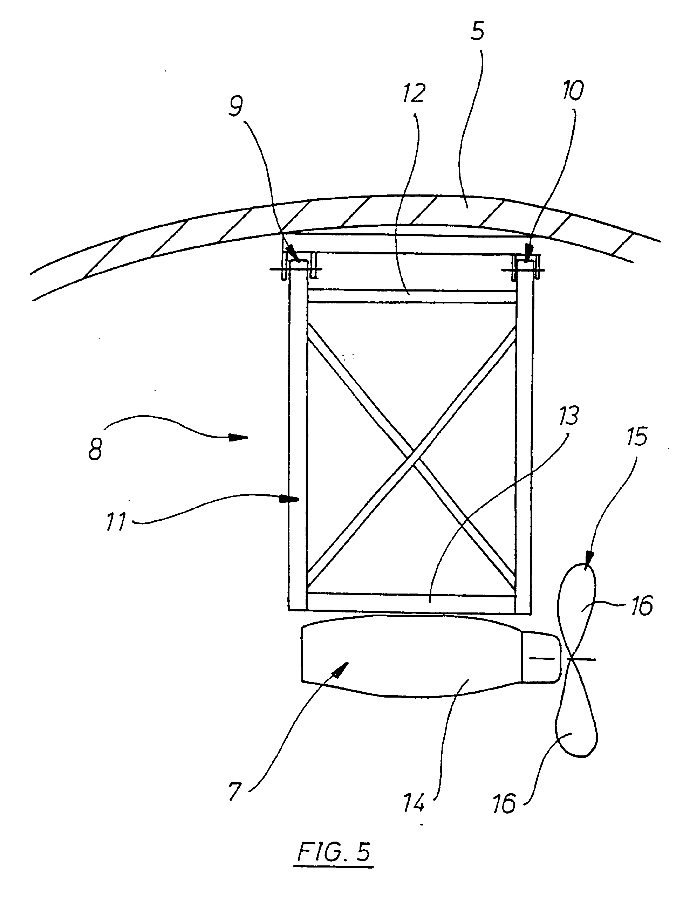

Wie dies insbesondere auch aus den Fig. 4 und 5 ersichtlich ist, die vergrößerte Detaildarstellungen bzw. eine Draufsicht auf die Ausführungsform gemäß Fig. 1 zeigen, ist ferner ein Tauchrührgerät 7 vorgesehen, das im Fermenterbehälter 3 über eine Schwingenanordnung 8 im Fermenterbehälter in einem Bereich unterhalb der Fermenterdeckenwand 6 schwenkbar angelenkt ist.

Wie dies insbesondere aus der Fig. 5 ersichtlich ist, die eine Draufsicht auf die Schwingenanordnung 8 mitsamt Tauchrührgerät 7 zeigt, umfasst die Schwingenanordnung 8 einen Schwingenarm 11, der mittels zweier beabstandeter, koaxial angeordneter Schwingen-Gelenklager 9, 10 schwenkbar an der Fermenterseitenwand 5 gelagert ist.

Der Schwingenarm 11 ist als in etwa rechteckiger Schwingenrahmen ausgebildet, wobei ein Rahmenteil 12 an den beiden Schwingen-Gelenklager 9, 10 schwenkbar gelagert ist und an einem dem Rahmenteil 12 gegenüberliegenden Rahmenteil 13 das Tauchrührgerät 7 befestigt ist.

Das Tauchrührgerät 7 weist einen zylindrischen Elektrodrehantrieb 14 auf, der mit dem Rahmenteil 13 in einer Parallelanordnung verbunden ist, wobei als Rührelement ein Rührpropeller 15 mit hier beispielhaft zwei Rührflügeln 16 vorgesehen ist und diese Rührflügel 16 den Schwingenarm 11 seitlich überragen.

Wie dies aus der Fig. 4 ersichtlich ist, ist ein Elektrokabel 17 zum Elektrodrehantrieb 14 des Tauchrührgeräts 7 von außen her dicht in den Fermenterbehälter 3 eingeführt und ortsfest bis zu einem der Schwingen-Gelenklager 9, 10 sowie anschließend befestigt am Schwingenarm 11 zum Elektrodrehantrieb 14 geführt.

Das Tauchrührgerät 7 ist von außerhalb des Behälters über eine Betätigungseinrichtung 18 in unterschiedliche Höhenpositionen ein- und feststellbar, wie dies sowohl in der Fig. 1 als auch in der Fig. 4 mit durchgezogenen Linien sowie strichliert eingezeichnet ist. Diese Betätigungseinrichtung 18 (Fig. 4) umfasst ein mit dem Schwingenarm 11 gelenkig verbundenes und über eine Dichtmanschette 19 dicht in den Außenbereich des Fermenterbehälters 3 geführtes Stellgestänge 20, das aufgrund der gelenkigen Anlenkung am Schwingenarm 11 ein Verschwenken des Schwingenarmes 11 und damit des Tauchrührgerätes 7 in unterschiedliche Höhenpositionen ermöglicht, wie dies hier lediglich schematisch in der Fig. 4 dargestellt ist.

Aus der Fig. 1 ist ersichtlich, dass die Schwingen-Gelenklager 9, 10 so anzuordnen sind und dass die Länge des Schwingenarms 11 so zu wählen ist, dass eine Verstellung des Tauchrührgerätes 7 in eine in der Fig. 1 und in der Fig. 4 mit durchgezogenen Linien dargestellte Wartungsposition 21 unmittelbar in den Bereich einer Wartungsöffnung 22 in der Fermenterdeckenwand 6 durchführbar ist. Diese Wartungsöffnung 22 kann mit einer Abdeckung 23, die in der Fig. 1 lediglich äußerst schematisch dargestellt ist, dicht verschlossen werden.

Der Fermenterbehälter 3 kann grundsätzlich eine Zylinderform aufweisen, so dass eine zylindrische Fermenterseitenwand 5 vorgesehen ist, wie dies aus der Fig. 5 ersichtlich ist. Alternativ dazu kann der Fermenterbehälter 3 jedoch auch kastenförmig mit in etwa geraden Fermenterseitenwänden ausgebildet sein.

In der Darstellung der Fig. 2 ist eine alternative Ausführungsform zu dem Aufbau gemäß Fig. 1 gezeigt, bei dem das oder die Schwingengelenklager 24 im Bereich unterhalb der Fermenterdeckenwand 6 in einem mittleren Bereich derselben angeordnet sind, wobei das Tauchrührgerät 7 hier in einer Wartungsposition 25 in einen seitlichen Bereich der Fermenterdeckenwand 6 und einer dort ausgebildeten Wartungsöffnung 26 verschwenkt werden kann. Wie dies in der Fig. 2 ferner strichliert und schematisch eingezeichnet ist, kann auf der gegenüberliegenden Seite alternativ oder aber auch zusätzlich eine weitere Wartungsöffnung 27 in der Fermenterdeckenwand 6 ausgebildet sein, so dass das Tauchrührgerät alternativ in die Wartungsposition 25 oder in eine Wartungsposition 28 verschwenkt werden kann.

Bei einer derartigen Anordnung des Tauchrührgeräts wird, wie ein Vergleich mit der Ausführungsform gemäß Fig. 1 zeigt, ein Verstellen des Tauchrührgeräts 7 von der Mitte aus in der Bildebene sowohl nach rechts als auch nach links ermöglicht, so dass hier ein insgesamt größerer Verstellweg im Fermenterbehälter 3 zur Verfügung gestellt wird.

Gemäß einer weiteren alternativen Ausführungsform entsprechend der Fig. 3 kann ferner auch ein Schwingen-Gelenklager 29 in einem mittleren Höhenbereich der Fermenterseitenwand 5 angeordnet sein, wobei dann hier das Tauchrührgerät 7 ebenfalls zu einer in einem seitlichen Fermenterdeckenwandbereich ausgebildeten Wartungsöffnung 30 in eine Wartungsposition 31 verschwenkt werden kann. Auch hier ist ein größerer Verschwenkweg gegeben, da das Tauchrührgerät um das oder die Schwingen-Gelenklager 29 sowohl nach oben als auch nach unten verschwenkt werden kann. Im dargestellten großen Fermenterbehälter 3 der Fig. 3 ist es für gute Rührergebnisse vorteilhaft ein zweites, gegenüberliegendes Tauchrührgerät 7a zu verwenden. Dessen Schwingen-Gelenklager 29a ist verschiebbar an einer vertikalen Höhenstellschiene 32 angeordnet und über ein schematisch eingezeichnetes Betätigungsseil 33 höhenverstellbar gehalten.

## Patentansprüche

1. Biogasanlage zur Fermentation von organischen Stoffen,
mit einem Fermenter, der einen Fermenterbehälter mit einer Fermenterbodenwand, mit Fermenterseitenwänden und mit einer Ferenterdeckenwand sowie wenigstens ein Tauchrührgerät umfasst, wobei
das wenigstens eine Tauchrührgerät im Fermenterbehälter an einer Halte- und Stelleinrichtung befestigt und von außerhalb des Behälters über eine Betätigungseinrichtung in unterschiedlichen Höhenpositionen ein- und feststellbar ist,
**dadurch gekennzeichnet,**
**dass** die Halte- und Stelleinrichtung als Schwingenanordnung (8) ausgebildet ist,
**dass** dazu wenigstens ein Schwingen-Gelenklager (9, 10; 24; 29) mit etwa horizontaler Gelenklagerachse ortsfest im Fermenterbehälter (3) angebracht ist,
**dass** mit dem Schwingen-Gelenklager (9, 10; 24; 29) ein Schwingenarm (11) schwenkbar verbunden ist, und
**dass** das Tauchrührgerät (7) im Bereich des freien Schwingenarmendes mit diesem verbunden ist, so dass bei unterschiedlichen Schwenkpositionen des Schwingenarms (11) entsprechend zugeordnete, unterschiedliche Höhenpositionen des Tauchrührgeräts (7) einstellbar sind.

2. Biogasanlage nach Anspruch 1, **dadurch gekennzeichnet,**
**dass** das Tauchrührgerät (7) einen Drehantrieb (14) als elektrischen oder hydraulischen oder pneumatischen Antrieb und wenigstens ein drehangetriebenes Rührelement (15) aufweist, und
**dass** das Tauchrührgerät (7) so am Schwingenarm (11) angebracht ist, dass die Rührelement-Drehachse achsparallel zur Gelenklagerachse verläuft.

3. Biogasanlage nach Anspruch 1 oder 2, **dadurch gekennzeichnet,**
**dass** der Schwingenarm (11) als etwa rechteckiger Schwingenrahmen ausgebildet ist,
**dass** ein Rahmenteil (12) an zwei beabstandeten, koaxial angeordneten Schwingen-Gelenklagern (9, 10) schwenkbar gelagert ist, und
**dass** am gegenüberliegenden Rahmenteil (13) das Tauchrührgerät (7) befestigt ist.

4. Biogasanlage nach Anspruch 3, **dadurch gekennzeichnet,**
**dass** das Tauchrührgerät (7) einen zylindrisch geformten Drehantrieb (14) aufweist, der mit dem besagten, gegenüberliegenden Rahmenteil (13) in einer Parallelanordnung verbunden ist, und
**dass** als Rührelement ein Rührpropeller (15) mit wenigstens zwei Rührflügeln (16) verwendet ist, wobei die Rührflügel (16) den Schwingenrahmen (11) seitlich überragen.

5. Biogasanlage nach einem der Ansprüche 1 bis 3, **dadurch gekennzeichnet, dass** eine Versorgungsleitung (17) zu einem Drehantrieb (14) des Tauchrührgeräts (7) von außen dicht in den Fermenterbehälter (3) eingeführt und ortsfest bis zum wenigstens einen Schwingen-Gelenklager (9, 10; 24; 29) mit einer dortigen Schwenkmöglichkeit und anschließend befestigt am Schwingenarm (11) zum Drehantrieb (14) geführt ist.

6. Biogasanlage nach einem der Ansprüche 1 bis 5, **dadurch gekennzeichnet, dass** das wenigstens eine Schwingen-Gelenklager (9, 10; 24) in einem Bereich unmittelbar unter der Fermenterdeckenwand (6) angebracht ist.

7. Biogasanlage nach Anspruch 6, **dadurch gekennzeichnet, dass** das Schwingen-Gelenklager (9, 10) im Bereich einer Fermenterseitenwand (5) mit einer etwa parallel dazu verlaufenden Gelenklagerachse angebracht ist.

8. Biogasanlage nach einem der Ansprüche 1 bis 7, **dadurch gekennzeichnet,**
**dass** das wenigstens eine Schwingen-Gelenklager (9, 10; 24; 29) so angeordnet ist und die Länge des Schwingenarms (11) so gewählt ist, dass eine Verstellung des Tauchrührgeräts (7) in eine Wartungsposition (21; 25, 28; 31) unmittelbar in den Bereich der Fermenterdeckenwand (6) durchführbar ist, und
**dass** im Bereich der Wartungsposition (21; 25, 28; 31) des Tauchrührgeräts (7) eine dicht verschließbare Wartungsöffnung (22; 26, 27; 30) in der Fermenterdeckenwand (6) angebracht ist.

9. Biogasanlage nach einem der Ansprüche 1 bis 8, **dadurch gekennzeichnet, dass** die Betätigungseinrichtung (18) aus einem mit dem Schwingenarm (11) und/oder dem Tauchrührgerät (7) gelenkig verbundenen und dicht über eine Dichtmanschette (19) in den Außenbereich des Fermenterbehälters (3) geführten Stellgestänge (20) besteht.

10. Biogasanlage nach einem der Ansprüche 1 bis 8, **dadurch gekennzeichnet, dass** die Betätigungseinrichtung ein Betätigungsseil umfasst, welches mit dem Schwingenarm und/oder dem Tauchrührgerät verbunden und über Umlenk- und Dichtelemente dicht in den Außenbereich des Fermenterbehälters geführt ist.

11. Biogasanlage nach einem der Ansprüche 1 bis 10, **dadurch gekennzeichnet,**
**dass** das Schwingen-Gelenklager (29a) an einer vertikalen, an der Innenseite einer Fermenterseitenwand (5) befestigten Höhenstellschiene (32) verschiebbar angebracht ist, und
**dass** eine Stellbetätigungseinrichtung an der Außenseite des Fermenterbehälters vorgesehen und mit einem Stellmittel (33) mit dem Schwingen-Gelenklager (29a) verbunden ist.

## Claims

1. Biogas-producing installation for the fermentation of organic materials,
with a fermenter which comprises a fermenter tank with a fermenter bottom wall, with fermenter side walls and with a fermenter top wall, and at least one submersible stirrer,
the at least one submersible stirrer in the fermenter tank being fastened to a holding and adjusting device and being able to be set and fixed in different height positions from outside the tank via an actuating device,
**characterized**
**in that** the holding and adjusting device is designed as a rocker arrangement (8),
**in that** for this purpose at least one rocker-type pivoting bearing (9, 10; 24; 29) with an approximately horizontal pivoting-bearing axis is fitted in a fixed position in the fermenter tank (3),
**in that** a rocker arm (11) is connected pivotably to the rocker-type pivoting bearing (9, 10; 24; 29), and
**in that** the submersible stirrer (7) is connected in the region of the free rocker-arm end to the latter, so that, at different pivoting positions of the rocker arm (11), correspondingly assigned, different height positions of the submersible stirrer (7) can be set.

2. Biogas-producing installation according to Claim 1, **characterized**
**in that** the submersible stirrer (7) has a rotary drive (14) as electric or hydraulic or pneumatic drive, and at least one rotationally driven stirring element (15), and
**in that** the submersible stirrer (7) is fitted to the rocker arm (11) in such a manner that the axis of rotation of the stirring element runs in an axially parallel manner to the pivoting-bearing axis.

3. Biogas-producing installation according to Claim 1 or 2, **characterized**
**in that** the rocker arm (11) is designed as an approximately rectangular rocker frame,
**in that** one frame part (12) is mounted pivotably on two spaced-apart, coaxially arranged rocker-type pivoting bearings (9, 10), and
**in that** the submersible stirrer (7) is fastened to the opposite frame part (13).

4. Biogas-producing installation according to Claim 3, **characterized**
**in that** the submersible stirrer (7) has a cylindrically shaped rotary drive (14) with is connected to the said opposite frame part (13) in a parallel arrangement, and
**in that** a stirring propeller (15) with at least two stirring blades (16) is used as the stirring element, the stirring blades (16) protruding laterally over the rocker frame (11).

5. Biogas-producing installation according to one of Claims 1 to 3, **characterized in that** a supply line (17) to a rotary drive (14) of the submersible stirrer (7) is introduced from the outside in a leak-proof manner into the fermenter tank (3) and is guided in a fixed position as far as the at least one rocker-type pivoting bearing (9, 10; 24; 29) with a possibility of pivoting there and subsequently, fastened to the rocker arm (11), is guided to the rotary drive (14).

6. Biogas-producing installation according to one of Claims 1 to 5, **characterized in that** the at least one rocker-type pivoting bearing (9, 10; 24) is fitted in a region directly below the fermenter top wall (6).

7. Biogas-producing installation according to Claim 6, **characterized in that** the rocker-type pivoting bearing (9, 10) is fitted in the region of a fermenter side wall (5) with a pivoting-bearing axis running approximately parallel thereto.

8. Biogas-producing installation according to one of Claims 1 to 7, **characterized**
**in that** the at least one rocker-type pivoting bearing (9, 10; 24; 29) is arranged in such a manner and the length of the rocker arm (11) is selected in such a manner that an adjustment of the submersible stirrer (7) into a maintenance position (21; 25, 28; 31) can be carried out directly in the region of the fermenter top wall (6), and
**in that,** in the region of the maintenance position (21; 25, 28; 31) of the submersible stirrer (7), a maintenance opening (22; 26, 27; 30), which is closable in a leak-proof manner, is provided in the fermenter top wall (6).

9. Biogas-producing installation according to one of Claims 1 to 8, **characterized in that** the actuating device (18) comprises an adjusting linkage (20) which is connected to the rocker arm (11) and/or the submersible stirrer (7) in an articulated manner and is guided in a leak-proof manner via a sealing sleeve (18) into the outer region of the fermenter tank (3).

10. Biogas-producing installation according to one of Claims 1 to 8, **characterized in that** the actuating device comprises an actuating cable which is connected to the rocker arm and/or the submersible stirrer and is guided via deflecting and sealing elements in a leak-proof manner into the outer region of the fermenter tank.

11. Biogas-producing installation according to one of Claims 1 to 10, **characterized**
**in that** the rocker-type pivoting bearing (29a) is fitted displaceably on a vertical height-adjusting rail (32) fastened to the inside of a fermenter side wall (5), and
**in that** an adjustment-actuating device is provided on the outside of the fermenter tank and is connected to the rocker-type pivoting bearing (29a) by an adjusting means (33).

## Revendications

1. Installation de biogaz pour la fermentation de matières organiques,
avec un fermentateur, qui comprend un récipient de fermentateur avec une paroi de fond de fermentateur, avec des parois latérales de fermentateur et avec une paroi de recouvrement de fermentateur ainsi qu'au moins un agitateur plongeur, à l'occasion de quoi
au moins un agitateur plongeur est fixé dans le récipient de fermentateur sur un dispositif de maintien et de réglage et peut être réglé et vérifié en dehors du récipient par l'intermédiaire d'un dispositif de commande dans différentes positions de hauteur,
**caractérisée en ce que**
le dispositif de maintien et de réglage est réalisé sous la forme d'un montage oscillant (8),
**en ce qu'**au moins un appui articulé oscillant (9, 10 ; 24 ; 29) avec un axe d'appui articulé à peu près horizontal est placé stationnaire dans le récipient de fermentateur (3),
**en ce qu'**un bras oscillant (11) est relié en étant pivotant à l'appui articulé oscillant (9, 10 ; 24 ; 29), et
**en ce que** l'agitateur-plongeur (7), dans la zone de l'extrémité libre du bras oscillant est relié à cette dernière, de sorte, qu'en cas de différentes positions d'articulation du bras pivotant (11), il est possible d'ajuster, en correspondance et associées, différentes positions en hauteur de l'agitateur-plongeur (7).

2. Installation de biogaz conformément à la revendication 1, **caractérisée en ce que**
L'agitateur-plongeur (7) présente un entraînement en rotation (14) sous forme d'entraînement électrique ou hydraulique ou pneumatique et au moins un élément agitateur entraîné en rotation (15), et
**en ce que** l'agitateur-plongeur (7) est disposé sur le bras pivotant (11) de sorte que l'axe de rotation de l'élément agitateur s'étend parallèlement à l'axe par rapport à l'axe de l'appui articulé.

3. Installation de biogaz conformément à la revendication 1 ou 2, **caractérisée en ce que** le bras pivotant (11) est réalisé sous la forme d'un cadre oscillant sensiblement rectangulaire,
**en ce qu'**une partie du cadre (12) est montée entraînable en pivotement sur deux appuis articulés oscillants (9, 10) disposés coaxialement et à distance, et
**en ce que** l'agitateur-plongeur (7) est fixé sur la partie du cadre opposée (13).

4. Installation de biogaz conformément à la revendication 3, **caractérisée en ce que**
L'agitateur-plongeur présente un entraînement à rotation (14) de forme cylindrique, qui est relié à la susdite partie de cadre opposée (13) dans un montage parallèle, et **en ce qu'**un agitateur à hélice (15) est utilisé en tant qu'élément agitateur avec au moins deux ailes agitatrices (16), à l'occasion de quoi les ailes agitatrices (16) font saillie latéralement du cadre oscillant (11).

5. Installation de biogaz conformément à l'une des revendications 1 à 3, **caractérisée en ce qu'**une conduite d'alimentation (17) d'un entraînement en rotation (14) de l'agitateur-plongeur (7) est introduite de l'extérieur de manière étanche dans le récipient du fermentateur (3), et est guidée solidairement jusqu'à au moins un appui articulé oscillant (9, 10 ; 24 ; 29) avec une possibilité locale de pivotement et est finalement fixée sur l'arbre pivotant (11) de l'entraînement en rotation (14).

6. Installation de biogaz conformément à l'une des revendications 1 à 5, **caractérisée en ce qu'**au moins un appui articulé oscillant (9, 10 ; 24) est placé dans un secteur situé directement en dessous de la paroi de recouvrement du fermentateur (6)

7. Installation de biogaz conformément à la revendication 6, **caractérisée en ce que** l'appui articulé oscillant (9 ,10) est placé dans le secteur d'une paroi latérale du fermentateur (5) avec un axe d'appui articulé s'y étendant à peu près parallèlement.

8. Installation de biogaz conformément à l'une des revendications 1 à 7, **caractérisée en ce que**
au moins un appui articulé rotatif (9, 10 ; 24 ; 29) est disposé, et la longueur du bras pivotant (11) est choisie, de telle sorte qu'un déplacement de l'agitateur plongeur (7) peut être réalisé dans une position de maintenance (21 ; 25, 28 ; 31) directement dans le secteur de la paroi de recouvrement du fermentateur (6), et
**en ce que** dans la zone de la position de maintenance (21 ; 25, 28 ; 31) de l'agitateur-plongeur (7), une ouverture de maintenance pouvant être fermée de manière étanche (22 ; 26, 27 ; 30) est placée dans la paroi de recouvrement du fermentateur (6).

9. Installation de biogaz conformément à l'une des revendications 1 à 8 **caractérisée en ce que** le dispositif de commande (18) est composé d'une tige de réglage (20) reliée artculée au bras pivotant (11) et / ou à l'agitateur-plongeur (7) et guidée de manière étanche via une manchette d'étanchéité (19) dans la zone extérieure du récipient du fermentateur (3).

10. Installation de biogaz conformément à l'une des revendications 1 à 8 **caractérisée en ce que** le dispositif de commande comprend un câble d'actionnement qui est raccordé au bras pivotant et / ou à l'agitateur-plongeur et qui est guidé via les éléments de déviation et d'étanchéité de manière étanche dans la zone extérieure du récipient du fermentateur.

11. Installation de biogaz conformément à l'une des revendications 1 à 10 **caractérisée en ce que**
l'appui articulé oscillant (29a) est installé déplaçable sur un rail à réglage en hauteur (32) vertical fixé sur le côté intérieur d'une paroi latérale de fermentateur (5), et
qu'un dispositif de commande de réglage est prévu sur le côté extérieur du récipient de fermentateur et relié à l'appui articulé oscillant (29a) avec un moyen de réglage (33).
